# FASCICULE DE BREVET EUROPEEN

(11) **EP 2 065 634 B1**
(45) Date de publication et mention de la délivrance du brevet: **30.08.2017**
(21) Numéro de dépôt: 08170120.3
(22) Date de dépôt: 27.11.2008
(51) Int. Cl.: F21S 8/00, F21V 5/00, F21V 5/02, F21V 21/40, F21V 23/04, H05B 33/08, A61B 90/30, F21Y 101/00, F21Y 105/10, F21Y 115/10, F21W 131/205

(54) **Dispositif d'éclairage médical**
Medizinische Beleuchtungsvorrichtung
Medical lighting device

(30) Priorité: 27.11.2007 FR 0759341
(43) Date de publication de la demande: 03.06.2009
(73) Titulaire: Surgiris, 59000 Lille (FR)
(72) Inventeur: Papin, Denis, 59970 Fresnes sur Escaut (FR)
(74) Mandataire: Balesta, Pierre

(56) Documents cités:
- EP-A- 1 433 998
- WO-A-02/06723
- US-A- 4 196 460
- US-A- 4 288 844
- US-A1- 2003 165 055
- US-A1- 2005 231 945

## Description

La présente invention a pour objet un dispositif d'éclairage médical ou chirurgical. Elle trouve notamment son application à l'éclairage médical ou chirurgical dans un bloc opératoire.

Un des paramètres important à prendre en compte pour un tel dispositif d'éclairage est la focalisation de l'éclairage. Il est en effet important de pouvoir régler le diamètre de la tâche d'éclairement en fonction des circonstances d'utilisation.

On connaît des dispositifs d'éclairage médical comprenant un module d'éclairage avec une ou plusieurs sources lumineuses, et dans lesquels, pour faire varier le diamètre de la tâche d'éclairement, on fait varier la distance entre les sources lumineuses et la zone à éclairer.

Un des problèmes posés par ces dispositifs est qu'ils nécessitent l'utilisation de moyens mécaniques pour faire varier la distance entre les sources lumineuses et la zone à éclairer. Ces moyens mécaniques induisent alors des contraintes en terme de conception (complexité mécanique), fabrication (réglage et mise en service) et utilisation (usure, nécessité de procéder à des réglages périodiques).

Un autre problème posé par ces dispositifs est la perte de puissance lors de l'augmentation du diamètre de la tâche d'éclairement. En effet, pour augmenter ce diamètre, on augmente la distance entre les sources lumineuses et la zone à éclairer, d'où cette perte de puissance.

On connaît, par exemple des documents EP 1 722 156 et EP 1 568 938 / US 2005/0231945, des dispositifs dans lesquels le problème précité de la perte de puissance est réglé.

Les dispositifs décrits dans ces documents comprennent plusieurs modules, chacun comprenant plusieurs sources lumineuses.

Les différents modules sont généralement agencés en nid d'abeille, autour d'un module central, et articulés mécaniquement par rapport à ce module central en sorte de pouvoir être inclinés par rapport à celui-ci.

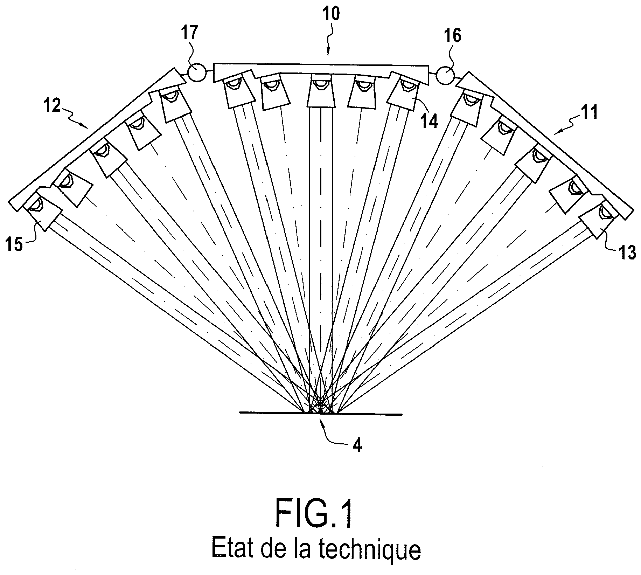

On a ainsi représenté schématiquement à la figure 1 l'exemple d'un tel dispositif de l'état de la technique, avec un module central 10 et deux modules périphériques 11 et 12, chacun comprenant plusieurs sources lumineuses 13, 14 et 15 de type LED.

Le module périphérique 11 est articulé par rapport au module central 10 au niveau d'un axe de rotation 16.

Le module périphérique 12 est quant à lui articulé par rapport au module central 10 au niveau d'un axe de rotation 17.

Avec de tels dispositifs, la variation du diamètre de la tâche d'éclairement 4 est obtenue par variation de l'inclinaison des modules périphériques 11 et 12 par rapport au module central 10.

Dans le cas de systèmes à base de lampes halogènes et de réflecteurs, le réglage s'effectue par un mouvement de la lampe suivant l'axe du réflecteur.

Comme indiqué plus haut, ces dispositifs résolvent le problème de la perte de puissance lors de l'augmentation du diamètre de la tâche d'éclairement, cette perte devenant faible puisque limitée à la remontée des modules périphériques par rotation (perte nulle sur le module central ; perte quasi-nulle pour les sources lumineuses des modules périphériques à proximité du module central ; perte maximale mais faible pour les sources lumineuses en périphérie des modules périphériques).

Toutefois, avec ces dispositifs, le problème lié à la présence de systèmes mécaniques, tel qu'exposé, plus haut persiste. Il est même accentué du fait que ces éléments mécaniques sont plus complexes et sophistiqués que ceux nécessaires à la simple translation verticale du dispositif, et du fait de la présence de plusieurs modules articulés.

On connaît enfin, notamment du document EP 1 722 157 / US 2006/0291204, des dispositifs semblables aux dispositifs précédents, mais dans lesquels les caractéristiques d'éclairement sont réglées électroniquement.

Ces dispositifs sont constitués de plusieurs modules, chacun comprenant plusieurs sources lumineuses, et agencés, généralement en nid d'abeille, autour d'un module central.

Chaque module est conçu pour permettre d'éclairer à lui seul le champ opératoire.

Les modules sont par ailleurs agencés en sorte qu'il n'y ait pas de rupture d'éclairage au passage d'un module à l'autre, pour que l'éclairage soit perçu comme uniforme.

Pour faciliter la circulation de l'air ventilé généralement depuis le plafond vers le bas, il est préférable que les modules soient espacés, ce qui augmente la complexité de l'agencement et du contrôle des sources lumineuses pour garantir cette uniformité d'éclairage.

Ces dispositifs comprennent également des moyens de contrôle destinés à contrôler individuellement l'intensité des sources lumineuses des différents modules.

Ces moyens de contrôles sont ainsi configurés pour permettre de définir différentes zones d'éclairage concentriques en fonction des modules activés. Cette configuration est prédéfinie et détermine des modes d'utilisation prédéfinis.

Ainsi, la plus petite zone d'éclairage correspond à l'activation du module central, par activation de toutes ses sources lumineuses.

La zone d'éclairage intermédiaire correspond, en plus de l'activation du module central, à l'activation d'une partie des modules périphériques.

Enfin, la zone d'éclairage la plus grande correspond à l'activation de tous les modules périphériques et du module central.

Ces dispositifs permettent de modifier la zone d'éclairage en fonction de la position du ou des chirurgiens, pour éviter d'éclairer des zones obstruées par la tête de ces chirurgiens.

Toutefois, un premier problème posé par ce type de dispositif est qu'il est constitué de plusieurs modules dont l'agencement génère toujours des problèmes mécaniques et de montage, et dont le contrôle est rendu complexe.

Il faut en effet toujours prévoir de régler l'inclinaison des modules périphériques, et la progression de la taille des zones éclairées se fait toujours en activant, en totalité ou partiellement, les modules lumineux du centre vers la périphérie, faute de quoi les zones éclairées obtenues ne sont pas pleines.

Un autre problème posé par ce type de dispositif est qu'il ne permet pas de régler la focalisation ou défocalisation, c'est-à-dire le diamètre de la tâche d'éclairement, autrement que par l'action mécanique d'inclinaison des modules latéraux.

En effet, avec de tels dispositifs, lorsqu'un mode d'éclairement prédéfini est sélectionné, il n'est pas possible de régler électroniquement de façon continue ou quasi-continue le diamètre de la tâche d'éclairement autrement qu'en sélectionnant un autre mode, et sans perte de puissance.

Il n'est par exemple pas possible de faire varier le diamètre du champ éclairé, sans perte de puissance, avec le seul module central. On connaît du document WO02/06723 un éclairage pour soins dentaires constitué d'un seul module et dont les sources lumineuses de type LED sont contrôlées individuellement, cependant le diamètre de la tâche d'éclairement de ce dispositif est fixé par construction. Le problème qui se pose alors est donc notamment de disposer d'un éclairage médical simple dans sa conception, sa fabrication et son utilisation, qui permet de faire varier le diamètre de la tâche d'éclairement sans perte de puissance.

L'objet de l'invention est donc d'apporter une solution aux problèmes précités parmi d'autres problèmes.

L'invention se rapporte ainsi à un dispositif d'éclairage médical ou chirurgical comprenant un module d'éclairage dans lequel sont disposées plusieurs sources lumineuses.

L'intensité de ces sources lumineuses est contrôlée individuellement par des moyens de contrôle.

Ces moyens de contrôle sont configurés en sorte que le champ éclairé prenne une forme pleine sensiblement circulaire, et forme donc un disque. Le diamètre du champ éclairé sera appelé diamètre de focalisation.

Par ailleurs, le dispositif selon la présente invention comprend également un élément optique disposé entre les sources lumineuses et la zone à éclairer.

Cet élément optique comprend un premier et un deuxième dioptre. Le premier dioptre est plan et orienté vers la zone à éclairer. Le deuxième dioptre est orienté vers les sources lumineuses et présente au moins deux facettes inclinées l'une par rapport à l'autre dont une au moins n'est pas parallèle au premier dioptre.

Cet élément optique agit comme autant de prisme que de couple premier dioptre-facette ; la configuration de cet élément optique permettant une mise en forme du faisceau prédéterminée ainsi que sa redirection.

Il est par ailleurs possible de disposer un nombre très important de sources lumineuses dans le module pour augmenter la résolution d'éclairage, l'élément optique permettant la redirection des faisceaux lumineux d'une façon prédéterminée.

Ainsi, les moyens de contrôles sont configurés en tenant compte de l'élément optique de sorte que le diamètre de focalisation varie par le contrôle sélectif des sources lumineuses et la redirection du ou des faisceaux lumineux émis par la ou les sources lumineuses, le tout à puissance lumineuse constante.

Le dispositif selon la présente invention permet avantageusement, avec un unique module d'éclairage, de faire varier le diamètre de la tâche d'éclairement (et donc le diamètre de focalisation) et de maintenir l'intensité lumineuse constante, ceci par le contrôle sélectif électronique des sources lumineuses configuré en adéquation avec la configuration de l'élément optique.

Dans une variante de réalisation, toutes les sources lumineuses sont orientées dans le même plan, en sorte que les rayons lumineux émergeant de ces sources lumineuses soient tous parallèles.

Il n'est ainsi pas nécessaire de prévoir une inclinaison quelconque des sources lumineuses, ce qui facilite la fabrication et l'installation.

Eventuellement, les moyens de contrôle peuvent être configurés en sorte de faire varier le spectre de lumière du champ éclairé par le contrôle sélectif des sources lumineuses. Ainsi, en fonction de l'intervention chirurgicale, il sera possible de choisir un spectre de lumière déterminé, par exemple pour mettre en évidence les différentes zones du champ opératoire (os, organes, tissus, etc.).

De préférence, les sources lumineuses sont des sources à flux modulable, par exemple de type LED.

Dans une autre variante, éventuellement en combinaison avec une ou plusieurs quelconques des précédentes, le dispositif comprend au moins un module d'éclairage latéral dans lequel sont disposées plusieurs sources lumineuses, et des moyens de contrôle destinés à contrôler individuellement l'intensité des sources lumineuses de ce module d'éclairage latéral.

Dans ce cas, le dispositif comprend des moyens de contrôle principal destinés à contrôler les moyens de contrôle du module d'éclairage et les moyens de contrôle du module d'éclairage latéral.

Ces moyens de contrôle principal sont configurés en sorte que le champ éclairé prenne une forme pleine sensiblement circulaire, dont le diamètre, dit diamètre de focalisation, varie par contrôle sélectif des sources lumineuses de chacun de ces modules d'éclairage, à puissance lumineuse constante.

Dans encore une autre variante, éventuellement en combinaison avec une ou plusieurs quelconques des précédentes, les moyens de contrôle comprennent une interface de réglage du diamètre de focalisation.

De préférence, cette interface de réglage du diamètre de focalisation comprend une poignée. Cette poignée pourra être située au centre du module d'éclairage. La rotation de cette poignée dans un sens ou un autre par rapport à un axe de rotation permet alors de réduire ou d'agrandir le diamètre de focalisation, à puissance lumineuse constante.

D'autres caractéristiques et avantages de l'invention apparaîtront plus clairement et de manière complète à la lecture de la description ci-après des variantes préférées de réalisation du dispositif lesquelles sont données à titre d'exemples non limitatifs et en référence aux dessins annexés suivants.
- figure 1 : représente schématiquement un exemple de dispositif de l'état de la technique, vue de côté,
- figures 2a, 2b : représentent schématiquement un exemple de dispositif selon l'invention, vue de côté, respectivement dans deux positions d'éclairage.

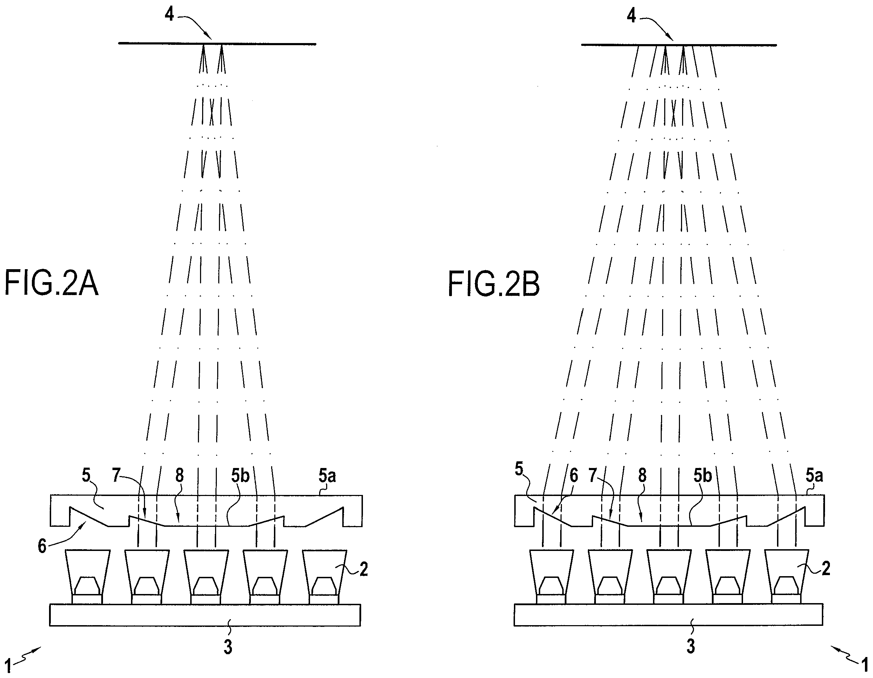

Aux figures 2a et 2b est donc représenté un exemple du dispositif d'éclairage médical selon l'invention.

Le dispositif comprend un module d'éclairage 1 lui-même comprenant plusieurs sources lumineuses 2 réparties de préférence de façon circulaire dans le module d'éclairage 1. Cette répartition n'apparaît bien évidemment pas aux figures 2a et 2b dans la mesure où le mode de représentation choisi est une vue de côté.

Ces sources lumineuses 2 sont de type LED, de préférence de forte puissance. Plus généralement, il pourra s'agir de sources lumineuses quelconques, de préférence à flux modulable.

Ces sources lumineuses 2 sont en nombre important dans le module d'éclairage 1.

Ces sources lumineuses sont contrôlées individuellement, notamment en ce qui concerne leur intensité, par des moyens de contrôle 3, par exemple de type circuit électrique ou électronique de commande, commandé par exemple par un microprocesseur.

Ainsi, ces moyens de contrôle 3 permettent de régler l'intensité de chacune des LED 2, entre une valeur nulle (LED éteinte) et une valeur maximale (intensité d'éclairage maximale).

Le réglage est déterminé en terme de pourcentage d'éclairement, et est destiné à faire varier le flux moyen émis par chaque LED, de préférence par une méthode de type PWM (« pulse width modulation ») entre 0 et la valeur maximale de courant.

Par différents regroupements appropriés des sources lumineuses 2, et par le réglage de l'intensité de chacune de ces sources lumineuses, opérés par les moyens de contrôle, on peut définir une tâche d'éclairement ou un champ éclairé 4 plein, circulaire et de diamètre variable.

La variation de diamètre de focalisation peut être encodée sur 8 bits pour traitement par le microprocesseur. Avec un tel encodage, on a donc 256 valeurs possibles, ce qui est suffisant pour assurer une continuité du réglage.

A chaque valeur de diamètre correspond une matrice de coefficients déterminant le flux émis par chaque LED ou sources lumineuses 2 du module d'éclairage 1, et donc le courant d'alimentation de celles-ci.

Idéalement, le programme de contrôle ainsi que les matrices de coefficients sont enregistrés dans une mémoire réinscriptible in situ, de type mémoire flash par exemple, afin de permettre une mise à jour par l'intermédiaire d'une liaison (par exemple de type USB ou RS232) avec un ordinateur.

De préférence, on prévoit une poignée de réglage du diamètre du champ éclairé, non représentée sur les figures, qui fait partie d'une interface de réglage du dispositif d'éclairage.

Cette interface comprendra généralement d'autres moyens de commande destinés à permettre à un utilisateur de régler différents paramètres de fonctionnement du dispositif d'éclairage, ces moyens de commandes pouvant être situés à proximité ou non de ladite poignée.

Cette poignée peut par exemple être située au centre du module d'éclairage 1, et prendre la forme d'un bouton libre en rotation autour d'un axe de rotation, tel qu'une molette de réglage de préférence suffisamment grosse pour pouvoir être saisie par la main du chirurgien.

Cette poignée peut également être disposée sur un support prévu à cet effet, distinct du module d'éclairage 1, la poignée restant cependant bien sûr reliée aux moyens de commande 3.

En tournant cette poignée dans un sens ou dans l'autre, on définit une valeur de réglage du diamètre du champ éclairé. Cette poignée est reliée aux moyens de contrôle 3 par le biais d'un contrôleur qui transpose les signaux en provenance de la poignée en des matrices de coefficients. A une position de la poignée correspond donc une matrice de coefficients des flux de chaque source lumineuse 2, donc un diamètre du champ éclairé.

Alternativement, la poignée peut être associée à uniquement deux positions de réglages entre une position neutre, l'une correspondant à l'agrandissement du diamètre du champ éclairé (défocalisation) et l'autre correspondant à la réduction du diamètre du champ éclairé (focalisation).

Dans ce cas, la rotation de cette poignée (associée à un dispositif de type électro-aimant) dans un sens ou dans l'autre entraîne la focalisation ou la défocalisation continue jusqu'à relâchement de la poignée qui reprend alors sa position neutre. La focalisation ou la défocalisation cesse donc au relâchement de la poignée, et le diamètre du champ éclairé est ainsi réglé.

Dans ce cas, le contrôleur auquel est reliée la poignée transpose les signaux d'incrémentation ou de décrémentation en provenance de la poignée, en des matrices de coefficients successives. Ces matrices successives sont utilisées par les moyens de contrôle pour agrandir ou réduire selon un pas à pas continu le diamètre du champ éclairé.

Ainsi, à la figure 2a, seules les sources lumineuses centrales sont activées par les moyens de contrôle 3, ce qui génère un champ éclairé 4 étroit d'un certain diamètre.

A la figure 2b, toutes les sources lumineuses sont activées, y compris les sources périphériques, ce qui génère un champ éclairé 4 large de diamètre supérieur à celui de la figure 2a.

Avec un nombre de sources lumineuses 2 arbitrairement grand, on peut faire ainsi varier le diamètre du champ éclairé 4 de façon quasi-continue.

Entre la zone à éclairer et les sources lumineuses 2 est disposé un élément optique 5.

Cet élément optique 5 comprend deux dioptres 5a et 5b. Le premier dioptre 5a est orienté vers la zone à éclairer et est plan. Le deuxième dioptre 5b est orienté vers les sources lumineuses 2 et présente plusieurs facettes inclinées 6, 7, 8.

Dans l'exemple représenté aux figures 2a et 2b, la facette centrale 8 est parallèle au premier dioptre 5a, de sorte que le couple facette centrale 8 - dioptre 5a constitue un prisme neutre, c'est-à-dire transmettant sans déviation le rayon lumineux issu de la source lumineuse qui lui est en regard.

Par contre, les facettes 6 et 7 sont inclinées, selon deux inclinaisons différentes, et non parallèles au dioptre 5a.

Ainsi, les couples facette 6 - dioptre 5a et facette 7 - dioptre 5a constituent deux prismes de déviation ou diffraction du rayon lumineux issu des sources lumineuses qui leurs sont en regard.

Si la configuration d'ensemble du module d'éclairage 1, et la répartition des sources lumineuses 2 dans ce module d'éclairage 1, sont sensiblement circulaires, les facettes 6 et 7 peuvent très bien être constituées de couronnes tronconiques, la facette centrale 8 étant alors un disque.

D'autres configurations non circulaires sont cependant possibles.

Par ailleurs, le bord du champ éclairé 4 n'est pas nécessairement éclairé par les sources lumineuses 2 se trouvant au bord du module d'éclairage 1. Le choix de l'élément optique 5 et des inclinaisons des facettes 6, 7, 8 du deuxième dioptre 5b permet notamment de rediriger les rayons lumineux quelque soit la source lumineuse 2 émettrice.

Il est généralement nécessaire de prévoir, entre chaque source lumineuse 2 et l'élément optique 5, un élément (non représenté aux figures 2a et 2b) de convergence du faisceau lumineux émis par cette source lumineuse 2.

Dans le cas où la source lumineuse est une LED, on peut par exemple utiliser un collimateur ou une lentille pour assurer cette convergence.

D'autres types de réflecteurs peuvent être utilisés, en fonction de la nature des sources lumineuses 2.

De préférence, comme représenté aux figures 2a et 2b, toutes les sources lumineuses 2 sont orientées dans un même plan.

Ainsi, les rayons lumineux issus de ces sources lumineuses 2, après éventuel passage dans les éléments de convergence précités, et avant passage dans l'élément optique 5, sont tous parallèles.

Si l'on n'utilise pas d'éléments de convergence, les sources lumineuses 2 présentent généralement un cône (de révolution) de diffusion de la lumière émise, avec un angle au sommet plus ou moins grand (de l'ordre de 180° pour des LED par exemple). Dans ce cas, on assimile le rayon lumineux issu de ces sources lumineuses 2 à l'axe central de révolution du cône de diffusion de la lumière diffusée par ces sources lumineuses 2.

Ici encore, le choix de l'élément optique 5 et des inclinaisons des facettes 6, 7, 8 du deuxième dioptre 5b permet notamment de rediriger les rayons lumineux quelque soit la position de la source lumineuse 2 émettrice.

Les moyens de contrôles 3 peuvent permettre également de régler le spectre de lumière, donc la température de couleur, du champ éclairé 4, toujours par contrôle sélectif des sources lumineuses 2.

Ces moyens de contrôle 3 peuvent aussi corriger l'effet des variations des caractéristiques des sources lumineuses dans le temps, par des algorithmes de contrôle du flux émis par chaque source lumineuse 2.

Il est possible de prévoir l'assemblage d'un module d'éclairage 1 central avec des modules d'éclairage latéraux.

Dans ce cas, chaque module d'éclairage, central ou latéral, dispose de ses moyens de contrôle 3 individuels destinés à piloter les sources lumineuses 2 individuellement.

Ces moyens de contrôle individuels, par exemple de type circuit électrique ou électronique de commande, pourront être commandés par exemple par un microprocesseur qui stocke son intelligence de commande ainsi que les matrices de coefficients telles qu'expliqué ci-dessus.

Chacun de ces moyens de contrôle individuels contrôlant le module d'éclairage 3 central ou un module d'éclairage latéral, sera par ailleurs contrôlé et piloté par des moyens de contrôles principaux.

Ces moyens de contrôle principaux seront également par exemple de type circuit électrique ou électronique de commande, commandés par exemple par un microprocesseur qui stocke l'intelligence de commande ainsi que les matrices de coefficients telles qu'expliqué ci-dessus relatives à chacun des différents modules d'éclairage, central ou latéral.

Tous les modules sont alors fixes les uns par rapport aux autre. Le réglage du diamètre du champ éclairé, ou de la focalisation, sans variation de puissance, se fait uniquement électroniquement.

A titre d'exemple, on peut envisager une configuration avec une module central d'éclairage à 42 sources lumineuses, seul ou associé à de deux à six modules latéraux à 62 sources lumineuses, permettant un champ éclairé de diamètre variant de 10 à 30 cm à 1 m de hauteur.

## Revendications

1. Dispositif d'éclairage médical, notamment adapté pour l'éclairage chirurgical, comprenant un module d'éclairage (1) dans lequel sont disposées plusieurs sources lumineuses (2), un élément optique (5) disposé entre lesdites sources lumineuses (2) et la zone à éclairer, et des moyens de contrôle (3) destinés à contrôler individuellement l'intensité desdites sources lumineuses (2),
dans lequel :
- d'une part, lesdits moyens de contrôle (3) sont configurés en sorte que le champ éclairé (4) prenne une forme pleine sensiblement circulaire, et
- d'autre part, ledit élément optique (5) comprend un premier dioptre (5a) plan et orienté vers cette dite zone à éclairer, et un deuxième dioptre (5b) orienté vers lesdites sources lumineuses (2) et présentant au moins deux facettes (6, 7, 8) inclinées l'une par rapport à l'autre dont une au moins n'est pas parallèle au premier dioptre (5),
**caractérisé en ce que** le diamètre du champ éclairé, dit diamètre de focalisation, varie par le contrôle sélectif desdites sources lumineuses (2), tout en maintenant l'intensité lumineuse constante.

2. Dispositif selon la revendication 1, **caractérisé en ce que** toutes les sources lumineuses (2) sont orientées dans le même plan, en sorte que les rayons lumineux émergeant de ces dites sources lumineuses (2) soient tous parallèles.

3. Dispositif selon l'une quelconque des revendications 1 à 2, **caractérisé en ce que** les moyens de contrôle (3) sont configurés en sorte de faire varier le spectre de lumière du champ éclairé (4) par contrôle sélectif desdites sources lumineuses (2).

4. Dispositif selon l'une quelconque des revendications 1 à 3, **caractérisé en ce que** les sources lumineuses (2) sont à flux modulable, de préférence de type LED.

5. Dispositif selon l'une quelconque des revendications 1 à 4, **caractérisé en ce qu'**il comprend au moins un module d'éclairage latéral dans lequel sont disposées plusieurs sources lumineuses, et des moyens de contrôle destinés à contrôler individuellement l'intensité desdites sources lumineuses.

6. Dispositif selon la revendication 5, **caractérisé en ce qu'**il comprend des moyens de contrôle principal destinés à contrôler les moyens de contrôle (3) du module d'éclairage (1) et les moyens de contrôle du module d'éclairage latéral, et configurés en sorte que le champ éclairé (4) prenne une forme pleine sensiblement circulaire, dont le diamètre, dit diamètre de focalisation, varie par contrôle sélectif des sources lumineuses de chacun desdits modules d'éclairage, tout en maintenant l'intensité lumineuse constante.

7. Dispositif selon l'une quelconque des revendications 1 à 6, **caractérisé en ce que** les moyens de contrôle (3) ou les moyens de contrôle principal comprennent une interface de réglage du diamètre de focalisation.

8. Dispositif selon la revendication 7, **caractérisé en ce que** l'interface de réglage du diamètre de focalisation comprend une poignée, de préférence située au centre du module d'éclairage (1), dont la rotation dans un sens ou un autre par rapport à un axe de rotation permet de réduire ou agrandir ledit diamètre de focalisation.

## Patentansprüche

1. Medizinische Beleuchtungsvorrichtung, die insbesondere für die Operationsbeleuchtung geeignet ist, umfassend ein Beleuchtungsmodul (1), in dem mehrere Lichtquellen (2) angeordnet sind, ein optisches Element (5), das zwischen den Lichtquellen (2) und dem zu beleuchtenden Bereich angeordnet ist, und Steuermittel (3), die dazu bestimmt sind, einzeln die Intensität der Lichtquellen (2) zu steuern, wobei:
- einerseits die Steuermittel (3) derart konfiguriert sind, dass das beleuchtete Feld (4) eine vollflächige, im Wesentlichen kreisförmige Form annimmt, und
- andererseits das optische Element (5) einen ersten Diopter (5a), der eben ist und in Richtung des zu beleuchtenden Bereiches ausgerichtet ist, und einen zweiten Diopter (5b) aufweist, der in Richtung der Lichtquellen (2) ausgerichtet ist und mindestens zwei Facetten (6, 7, 8) aufweist, die zueinander geneigt sind, wovon mindestens eine nicht parallel zu dem ersten Diopter (5) ist,
**dadurch gekennzeichnet, dass** der Durchmesser des beleuchteten Feldes, der sogenannte Fokusdurchmesser, durch das selektive Steuern der Lichtquellen (2) variiert, indem gleichzeitig die Lichtintensität konstant gehalten wird.

2. Vorrichtung nach Anspruch 1, **dadurch gekennzeichnet, dass** alle Lichtquellen (2) in der gleichen Ebene ausgerichtet sind, so dass alle Lichtstrahlen, die aus diesen Lichtquellen (2) austreten, parallel sind.

3. Vorrichtung nach einem der Ansprüche 1 bis 2, **dadurch gekennzeichnet, dass** die Steuermittel (3) derart konfiguriert sind, um das Lichtspektrum des beleuchteten Feldes (4) durch selektives Steuern der Lichtquellen (2) variieren zu lassen.

4. Vorrichtung nach einem der Ansprüche 1 bis 3, **dadurch gekennzeichnet, dass** die Lichtquellen (2) mit modulierbarem Strom, vorzugsweise vom Typ einer LED, sind.

5. Vorrichtung nach einem der Ansprüche 1 bis 4, **dadurch gekennzeichnet, dass** sie mindestens ein seitliches Beleuchtungsmodul, in dem mehrere Lichtquellen angeordnet sind, und Steuermittel aufweist, die dazu bestimmt sind, einzeln die Intensität der Lichtquellen zu steuern.

6. Vorrichtung nach Anspruch 5, **dadurch gekennzeichnet, dass** sie Hauptsteuermittel aufweist, die dazu bestimmt sind, die Steuermittel (3) des Beleuchtungsmoduls (1) und die Steuermittel des seitlichen Beleuchtungsmoduls zu steuern, und derart konfiguriert sind, dass das beleuchtete Feld (4) eine vollflächige, im Wesentlichen kreisförmige Form annimmt, deren Durchmesser, der sogenannte Fokusdurchmesser, durch selektives Steuern der Lichtquellen von jedem dieser Beleuchtungsmodule variiert, indem gleichzeitig die Lichtintensität konstant gehalten wird.

7. Vorrichtung nach einem der Ansprüche 1 bis 6, **dadurch gekennzeichnet, dass** die Steuermittel (3) oder die Hauptsteuermittel eine Schnittstelle zum Einstellen des Fokusdurchmessers aufweisen.

8. Vorrichtung nach Anspruch 7, **dadurch gekennzeichnet, dass** die Schnittstelle zum Einstellen des Fokusdurchmessers einen Griff aufweist, der vorzugsweise in der Mitte des Beleuchtungsmoduls (1) angeordnet ist, dessen Drehung in einer oder einer anderen Richtung in Bezug auf eine Drehachse ermöglicht, den Fokusdurchmesser zu verringern oder zu vergrößern.

## Claims

1. A medical lighting device, notably adapted for surgical lighting, comprising a lighting module (1) in which are positioned several light sources (2), an optical element (5) positioned between said light sources (2) and the area to be illuminated, and control means (3) intended to individually control the intensity of said light sources (2),
wherein:
- on the one hand, said control means (3) are configured so that the illuminated field (4) assumes a full substantially circular shape, and
- on the other hand, said optical element (5) comprises a first planar diopter (5a) and oriented towards this said area to be illuminated and a second diopter (5b) oriented towards said light sources (2) and having at least two facets (6, 7, 8) tilted relative to each other for which at least one is not parallel to the first diopter (5),
**characterized in that** the diameter of the illuminated field, a so-called focusing field, varies by the selective control of said light sources (2), while maintaining the light intensity constant.

2. The device according to claim 1, **characterized in that** all the light sources (2) are oriented in a same plane so that the light rays emerging from these said light sources (2) are all parallel.

3. The device according to any of claims 1 to 2, **characterized in that** the control means (3) are configured so as to vary the light spectrum of the illuminated field (4) by selectively controlling said light sources (2).

4. The device according to any of claims 1 to 3, **characterized in that** the light sources (2) are with a flux which may be modulated, preferably of the LED type.

5. The device according to any of claims 1 to 4, **characterized in that** it comprises at least one lateral illumination module in which are positioned several light sources and control means intended to individually control the intensity of said light sources.

6. The device according to claim 5, **characterized in that** it comprises main control means intended to control the control means (3) of the illumination module (1) and the control means of the lateral illumination module, and configured so that the illuminated field (4) assumes a full substantially circular shape, for which the diameter, a so-called focusing diameter, varies by selective control of the light sources of each of the illumination modules, while maintaining the light intensity constant.

7. The device according to any of claims 1 to 6, **characterized in that** the control means (3) or the main control means, comprise an interface for adjusting the focusing diameter.

8. The device according to claim 7, **characterized in that** the interface for adjusting the focusing diameter, comprises a handle, preferably located at the center of the illumination module (1), for which the rotation in one direction or the other gives the possibility of reducing or enlarging said focusing diameter.
